# FASCICULE DE BREVET EUROPEEN

(11) **EP 0 495 721 B1**
(45) Date de publication et mention de la délivrance du brevet: **28.06.1995**
(21) Numéro de dépôt: 92400115.9
(22) Date de dépôt: 16.01.1992
(51) Int. Cl.: A61K 38/00

(54) **Utilisation d'un polypeptide ayant l'activité de l'interleukine 2 humaine pour la préparation de compositions pharmaceutiques destinées au traitement des pneumothorax**
Verwendung eines Polypeptids mit humaner Interleukin-2 Aktivität zur Herstellung pharmazeutischer Zusammensetzungen zur Behandlung von Pneumothoraxen
Use of a polypeptide having human interleukin-2 activity for the preparation of pharmaceutical compositions for the treatment of pneumothorax

(30) Priorité: 17.01.1991 FR 9100488
(43) Date de publication de la demande: 22.07.1992
(73) Titulaire: ROUSSEL-UCLAF, 75007 Paris (FR)
(72) Inventeur: Brandely, Maud, F-75017 Paris (FR); Boutin, Christian, F-13005 Marseille (FR)
(74) Mandataire: Bourgouin, André

(56) Documents cités:
- EP-A- 0 353 150

## Description

La présente invention concerne l'utilisation d'un polypeptide ayant l'activité de l'interleukine 2 humaine pour la préparation de compositions pharmaceutiques destinées au traitement des pneumothorax.

L'IL2 qui est une lymphokine produite par les lymphocytes T activés, possède une activité immunomodulatrice et une activité antitumorale décrites par exemple par Fletcher, M et al. (Lymphokine Research 6 1987 47-57), activités qui comprennent en particulier la capacité d'initier la prolifération des lymphocytes T et l'induction de la cytotoxicité des cellules NK (natural killer) et des cellules LAK (lymphokine activated killer). Les nombreuses études pharmacologiques qui ont montré l'efficacité potentielle de l'IL2 seule ou associée avec des cellules LAK et éventuellement d'autres agents thérapeutiques dans la thérapie du cancer ou des maladies infectieuses sont passées en revue par Winkelhake J, L et al. (Pharmacol. Reviews 1990 42 1-28).

L'utilisation de l'IL2 comme agent anticancéreux ou anti-infectieux chez le malade a été réalisée et les résultats cliniques décrits ont montré l'efficacité de l'administration de l'IL2 soit seule à haute dose, soit associée avec des cellules LAK et éventuellement à d'autres agents thérapeutiques, dans certains types de cancers ou de maladies infectieuses.

Or la demanderesse vient d'obtenir de façon inattendue des résultats montrant que l'IL2 présente une activité dans le traitement du pneumothorax.

Le pneumothorax est une affection caractérisée par une irruption d'air dans la cavité pleurale c'est-à-dire dans l'espace compris entre les plèvres pariétale et viscérale. Il comprend essentiellement les pneumothorax spontanés compliquant éventuellement l'évolution d'une pneumopathie ou d'une bronchopathie sous-jacente et les pneumothorax provoqués, par exemple un pneumothorax compliquant les suites d'une thoracoscopie, les pneumothorax d'origine traumatique ou ceux survenant comme complication de biopsies pleurales ou pulmonaires. Ces différents types de pneumothorax évoluent de façon fréquente vers la chronicité.

Parmi les pneumothorax spontanés qui sont la cause la plus fréquente de pneumothorax chroniques, on peut distinguer les pneumothorax bénins qui surviennent généralement sur un poumon antérieurement sain et qui ont souvent une tendance à la récidive et les pneumothorax symptomatiques de désordres broncho-pulmonaires localisés ou généralisés.

Il n'existe pas de traitement unique établi, notamment dans le cas du pneumothorax spontané, en particulier en raison d'une variabilité de l'estimation du risque de rechute spontanée qui est compris entre 20 et 50 % et qui augmente ensuite généralement à chaque épisode. Selon 72 séries de patients dont les résultats publiés ont porté sur plus de 1100 cas de pneumothorax spontanés, les traitements capables de rétablir l'adhérence (ou symphyse) pleurale qui sont décrits avec un taux de récidive associé comprennent : le repos au lit (taux de rechute à 28 %), le drainage (taux de rechute à 21 %), l'administration de tétracycline (taux de rechute 20,8 %), la pulvérisation d'une colle de fibrine (taux de rechute à 15,2 %), le talcage pleural (taux de rechute à 6 %) et la thoracotomie (taux de rechute à 1,5 %). Parmi ces traitements, le talcage comporte un risque théorique de carcinogénicité dans le cas d'un traitement prolongé ou répété. La thoracotomie demeure par conséquent le traitement de référence étant donné son faible taux de récidive, mais elle est parfois associée à des complications par exemple infectieuses et à un certain taux de morbidité.

La présente invention concerne donc l'utilisation d'un polypeptide ayant l'activité de l'interleukine 2 humaine pour préparer une composition pharmaceutique destinée au traitement des pneumothorax.

On entend par polypeptide ayant l'activité de l'IL2 humaine, l'IL2 humaine naturelle, l'IL2 humaine recombinante c'est-à-dire obtenue par la technologie de l'ADN recombinant, par exemple comme cela est décrit par Taniguchi, T et al. Nature 1983 302 305-310 ou dans le brevet EP 91539 B, des allèles ou des dérivés de ces produits tels que décrits par exemple par Ju, C et al. J. Biol. Chem. 1987 262 5723-5731.

Les pneumothorax que concerne l'invention comprennent les pneumothorax spontanés et les pneumothorax provoqués par un traumatisme ou un geste invasif, diagnostiqués par radiographie du thorax.

L'invention a notamment pour objet l'utilisation caractérisée en ce que le pneumothorax est un pneumothorax chronique. Le pneumothorax chronique concerné par l'invention est, soit un pneumothorax spontané tel qu'un pneumothorax spontané bénin, notamment un pneumothorax spontané récidivant, ou tel qu'un pneumothorax spontané symptomatique, par exemple de pneumopathies ou bronchopathies chroniques, éventuellement diagnostiqué au cours d'une radiographie thoracique de routine chez un malade ayant des antécédents variés souvent graves, soit un pneumothorax provoqué.

Dans tous les cas, le caractère chronique est affirmé par exemple par la persistance d'un bullage après dix jours de drainage pleural ou d'un décollement après ce même délai.

La demanderesse décrit ci-après l'utilisation d'IL2 humaine dans le traitement de 4 malades atteints de pneumothorax chroniques dont l'efficacité est montrée par l'obtention d'une symphyse pleurale complète stable.

L'invention a particulièrement pour objet l'utilisation caractérisée en ce que le polypeptide ayant l'activité de l'IL2 humaine est une IL2 recombinante pure. Les compositions pharmaceutiques préparées selon l'invention renferment une IL2 humaine recombinante, des allèles ou des dérivés de celle-ci, telle que décrite ci-dessus, pour laquelle on utilise des techniques de purification connues de l'homme de métier, qui permettent de préparer des produits purs.

L'invention a plus particulièrement pour objet l'utilisation caractérisée en ce que l'IL2 est une IL2 recombinante non glycosylée sous forme réduite. L'IL2 non glycosylée utilisée est notamment celle ayant la séquence de l'IL2 naturelle à 133 aminoacides avec éventuellement une méthionine N-terminale supplémentaire, dont les 3 cystéines en position 58, 105 et 125 sont sous la forme réduite, manifestant une activité biologique comparable à celle de l'IL2 oxydée ayant la même séquence comportant un pont disulfure en position 58-105. Cette IL2 sous forme réduite est décrite dans la demande de brevet européen EP 0353150. Par forme réduite, on entend que les restes cystéines que contient l'IL2 renferment un groupement sulfhydryle libre dont la détermination est faite, par exemple, par spectrophotométrie avec la dithiodipyridine comme réactif des thiols. L'activité biologique est déterminée par mesure de la prolifération de lignées cellulaires leucémiques de souris dépendantes de l'IL2 CTLL-2, avec un test colorimétrique au sel de tétrazolium (Mossmann, T J. Immunol. Meth 1983 65 55-63). L'activité spécifique des IL2 recombinantes utilisées dans l'invention est au moins égale à 0,5x.10**⁷** U BRMP/mg, de préférence 1x10**⁷** U BRMP/mg. L'unité d'activité IL2 est définie comme la quantité qui produit 50 % de la réponse maximum dans le test. On utilise comme standard un échantillon "Biological Response Modifier Programm (BRMP) reference agent human IL2 (jurkat)" fourni par National Cancer Institute (NCI).

L'invention a spécialement pour objet l'utilisation caractérisée en ce que l'IL2 est sans une forme adaptée pour être administrée par voie intrapleurale à la dose de 0,2x10**⁶** U à 3x10**⁶** U par injection et plus spécialement celle caractérisée en ce que l'IL2 est administrée à la dose de 0,5 à 1x10**⁶** U.

L'invention a tout spécialement pour objet l'utilisation caractérisée en ce que l'IL2 est sans une forme adaptée pour être administrée de façon répétée au moins 1 fois, de préférence au moins 3 fois, à intervalle d'au moins 1 jour.

La dose administrée, la fréquence de l'injection et la durée du traitement varient en fonction de l'état du malade et en particulier de l'obtention de la symphyse pleurale et de l'absence de récidive immédiate du pneumothorax.

L'IL2 est comprise dans une composition pharmaceutique, de préférence lyophilisée en flacon-dose contenant de 0,05 à 2 mg de principe actif et que l'on reconstitue avec de l'eau distillée pour injection. La solution obtenue est immédiatement diluée à l'aide d'un soluté, par exemple le glucose à 5 % pour l'administration en perfusion intrapleurale.

Selon l'utilisation préférée de l'invention, l'IL2 est l'IL2 recombinante réduite ci-dessus dont un exemple de préparation pharmaceutique est donnée plus loin, la dose journalière est d'environ 1x10**⁶** U en perfusion lente intrapleurale que l'on répète à intervalle d'au moins 1 jour, de préférence au moins 3 fois, jusqu'à obtention d'une symphyse complète. La dose administrée au total au malade représente une quantité d'IL2 inférieure à 1 mg, soit inférieure de plus de 10 fois aux quantités d'IL2 habituellement administrées dans le traitement par exemple de cancers.

### EXEMPLE 1 : composition pharmaceutique pour perfusion.

On a réalisé une préparation pour injection par voie intraveineuse en perfusion de formule :

| | |
|---|---|
| IL2 réduite | 0,5 mg |
| acide citrique | 5,0 mg |
| mannitol | 50,0 mg |
| eau stérile | 1 ml |
| glucose à 5 % | 50 ml |

### EXEMPLE 2 : étude clinique dans le traitement de pneumothorax.

L'étude inclut 4 malades ayant un pneumothorax chronique radiologiquement diagnostiqué : les malades A, C et D sont atteints de pneumothorax spontané récidivant et le malade B est atteint de pneumothorax accidentel, pour lesquels un drainage pleural pendant au moins 9 jours, éventuellement de façon répétée sur une période allant jusqu'à 2 mois, a été pratiqué avec un suivi de la radiographie pulmonaire qui a montré l'échec de la symphyse ou la récidive du pneumothorax. Au décours du drainage sans efficacité, un traitement par l'IL2 a été initié.

Les compositions d'IL2 préparées selon l'invention permettent d'injecter de faibles doses d'IL2 que l'on augmente éventuellement de 200.000 unités à 1,2 millions d'unités (U) par jour et que l'on répète à intervalle d'au moins 1 jour, selon la réponse observée, en perfusion lente par exemple comprise entre 1 et 4 heures à travers un, éventuellement deux, drain intrapleural. On utilise la préparation décrite à l'exemple 1 selon les protocoles décrits ci-dessus. Les résultats sont jugés sur la cessation du bullage et la disparition de l'épanchement gazeux sur la radiographie du thorax.

### Malade A.

Le patient, âgé de 13 ans, est porteur d'une mucoviscidose et a un pneumothorax avec un décollement du poumon sur toute la périphérie, survenu lors d'un effort à la toux au cours d'une infection bronchique :

| Durée antérieure de drainage | Dose d'IL2 (U/jour) | Réponse observée à la radiographie |
|---|---|---|
| 2 mois | J1 200.000 | |
| | J3 400.000 | |
| | J4 | symphyse partielle |
| | J7 200.000 | symphyse totale |

Le pneumothorax n'a pas récidivé après 2 mois de suivi.

### Malade B.

La patiente, âgée de 22 ans, est atteinte d'une fibrose pulmonaire pour laquelle la réalisation d'une biopsie pulmonaire, à visée diagnostique, a entrainé l'apparition d'un pneumothorax :

| Durée antérieure de drainage | Dose d'IL2 (U/jour) | Réponse observée à la radiographie |
|---|---|---|
| 10 jours | J1 500.000 | |
| | J3 800.000 | symphyse partielle |
| | J5 | symphyse totale |

Le pneumothorax n'a pas récidivé après 14 jours de suivi.

### Malade C.

La patiente, âgée de 73 ans, est hospitalisée pour une poussée évolutive d'angiosarcome et dont une radiographie pulmonaire révèle un pneumothorax avec décollement droit du poumon :

| Durée antérieure de drainage | Dose d'IL2 (U/jour) | Réponse observée à la radiographie |
|---|---|---|
| 19 jours | J1 1.000.000 | |
| | J3 1.000.000 | symphyse partielle |
| | J6 | symphyse totale |

### Malade D.

Le patient est âgé de 22 ans, avec de nombreux antécédents dont une maladie de Hodgkin au stade IIIB. Une radiographie pulmonaire a révélé un pneumothorax avec décollement gauche partiel du poumon :

| Durée antérieure de drainage | Dose d'IL2 (U/jour) | Réponse observée à la radiographie |
|---|---|---|
| 11 jours | J1 850.000 | |
| | J3 1.200.000 | |
| | J5 | symphyse totale |
| | J9 | rechute avec bulles |
| | J11 1.000.000 | |
| | J16 1.000.000 | |
| | J17 1.000.000 | |
| | J18 | symphyse totale |
| | J20 1.000.000 | |
| | J22 1.000.000 | |
| | J23 | symphyse totale |
| | J24 1.000.000 | |
| | J27 | symphyse totale |

Les résultats montrent une réponse complète chez l'ensemble des malades traités par l'IL2 (4/4).

## Revendications

1. Utilisation d'un polypeptide ayant l'activité de l'interleukine 2 humaine pour préparer une composition pharmaceutique destinée au traitement des pneumothorax.

2. Utilisation selon la revendication 1 caractérisée en ce que le pneumothorax est un pneumothorax chronique.

3. Utilisation selon la revendication 2 caractérisée en ce que le polypeptide ayant l'activité de l'IL2 humaine est une IL2 recombinante pure.

4. Utilisation selon la revendication 3 caractérisée en ce que l'IL2 est une IL2 recombinante non glycosylée sous forme réduite.

5. Utilisation selon la revendication 3 ou 4 caractérisée en ce que l'IL2 est sous une forme adaptée pour être administrée par voie intrapleurale à la dose de 0,2x10**⁶** U à 3x10**⁶** U par injection.

6. Utilisation selon la revendication 5 caractérisée en ce que l'IL2 est sous une forme adaptée pour être administrée à la dose de 0,5 à 1 x10⁶ U.

7. Utilisation selon la revendication 6 caractérisée en ce que l'IL2 est sous une forme adaptée pour être administrée de façon répétée au moins 1 fois, de préférence au moins 3 fois, à intervalle d'au moins 1 jour.

## Claims

1. Use of a polypeptide having the activity of human interleukin 2 for preparing a pharmaceutical composition intended for the treatment of pneumothoraces.

2. Use according to claim 1 characterized in that the pneumothorax is a chronic pneumothorax.

3. Use according to claim 2 characterized in that the polypeptide having the activity of human interleukin 2 is a pure recombinant IL2.

4. Use according to claim 3 characterized in that the IL2 is a non-glycosylated recombinant IL2 in reduced form.

5. Use according to claim 3 or 4 characterized in that the IL2 is in a form suitable to be administered by interpleural route at a dose of 0.2 x 10⁶ U to 3 x 10⁶ U per injection.

6. Use according to claim 5 characterized in that the Il2 is in a form suitable to be administered at a dose of 0.5 to 1 x 10⁶ U.

7. Use according to claim 6 characterized in that the Il2 is in a form suitable to be administered in a repeated fashion at least once, preferably at least three times, at an interval of at least 1 day.

## Patentansprüche

1. Verwendung eines Polypeptids, das die Aktivität des menschlichen Interleukins 2 hat, zur Herstellung einer pharmazeutischen Zusammensetzung, die zur Behandlung von Pneumothorax bestimmt ist.

2. Verwendung gemäß Anspruch 1, dadurch gekennzeichnet, daß der Pneumothorax ein chronischer Pneumothorax ist.

3. Verwendung gemäß Anspruch 2, dadurch gekennzeichnet, daß das Polypeptid, das die Aktivität des menschlichen IL2 hat, ein reines, rekombinantes IL2 ist.

4. Verwendung gemäß Anspruch 3, dadurch gekennzeichnet, daß das IL2 ein nicht glykosiliertes, rekombinantes IL2 in reduzierter Form ist.

5. Verwendung gemäß Anspruch 3 oder 4, dadurch gekennzeichnet, daß das IL2 in einer Form ist, die angepasst ist, um auf intrapleuralem Wege in der Dosis von 0,2 x 10⁶ E bis 3 x 10⁶ E durch Injektion verabreicht zu werden.

6. Verwendung gemaß Anspruch 5, dadurch gekennzeichnet, daß das IL2 in einer angepassten Form ist, um in der Dosis von 0,5 bis 1 x 10⁶ E verabreicht zu werden.

7. Verwendung gemäß Anspruch 6, dadurch gekennzeichnet, daß das IL2 in einer angepassten Form ist, um wiederholt mindestens ein Mal, vorzugsweise mindestens drei Mal im Zeitraum von mindestens einem Tag verabreicht zu werden.
